# EUROPÄISCHE PATENTSCHRIFT

(11) **EP 0 934 315 B1**
(45) Veröffentlichungstag und Bekanntmachung des Hinweises auf die Patenterteilung: **13.06.2001**
(21) Anmeldenummer: 97912120.9
(22) Anmeldetag: 06.10.1997
(51) Int. Cl.: C07D 413/12, C07D 417/12, A01N 43/76, A01N 43/78

(54) **SULFONYLBENZAZOLONE**
SULFONYL BENZAZOLONES
SULFONYLBENZAZOLONES

(30) Priorität: 17.10.1996 DE 19642865
(43) Veröffentlichungstag der Anmeldung: 11.08.1999
(73) Patentinhaber: BAYER AG, 51368 Leverkusen (DE)
(72) Erfinder: ASSMANN, Lutz, D-23701 Eutin (DE); ELBE, Hans-Ludwig, D-42329 Wuppertal (DE); MARKERT, Robert, D-51065 Köln (DE); TIEMANN, Ralf, D-51375 Leverkusen (DE); STENZEL, Klaus, D-40595 Düsseldorf (DE)
(86) Internationale Anmeldenummer: EP9705473
(87) Internationale Veröffentlichungsnummer: WO9817665

(56) Entgegenhaltungen:
- DE-A- 2 101 150
- DE-A- 3 102 907
- PATENT ABSTRACTS OF JAPAN vol. 5, no. 94 (C-059), 19.Juni 1981 & JP 56 036474 A (HOKKO CHEM IND CO LTD), 9.April 1981,

## Beschreibung

Die vorliegende Erfindung betrifft neue Sulfonylbenzazolone, ein Verfahren zu ihrer Herstellung und deren Verwendung als Mikrobizide im Pflanzenschutz und im Materialschutz.

Es ist bereits bekannt geworden, daß bestimmte Sulfonylbenzazolone fungizide Eigenschaften besitzen (vgl. DE-A 2 101 150). So läßt sich z. B. 7-Nitro-2-oxo-5-trifluormethyl-benzothiazol-3-sulfonsäuredimethylamid zur Bekämpfung von Pilzen einsetzen. Die Wirksamkeit dieses Stoffs ist gut, läßt aber bei niedrigen Aufwandmengen in manchen Fällen zu wünschen übrig.

Außerdem werden in der DE - A 3 102 907 unter anderem auch eine Reihe von Sulfonylbenzazolonen mit fungizider Wirksamkeit beschrieben. Es werden aber keine Stoffe dieses Typs erwähnt, in denen das Stickstoffatom über eine Sulfonyl - Gruppe mit einem Heterocyclus verbunden ist.

Es wurden nun neue Sulfonylbenzazolone der Formel 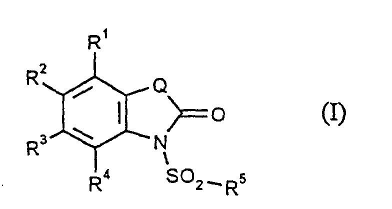 in welcher
- R¹, R², R³ und R⁴: unabhängig voneinander für Wasserstoff, Fluor, Chlor, Brom, Iod, Cyano, Nitro, geradkettiges oder verzweigtes Alkyl mit 1 bis 8 Kohlenstoffatomen, geradkettiges oder verzweigtes Halogenalkyl mit 1 bis 6 Kohlenstoffatomen und 1 bis 5 gleichen oder verschiedenen Halogenatomen, geradkettiges oder verzweigtes Alkoxy mit 1 bis 8 Kohlenstoffatomen, geradkettiges oder verzweigtes Halogenalkoxy mit 1 bis 6 Kohlenstoffatomen und 1 bis 5 gleichen oder verschiedenen Halogenatomen, geradkettiges oder verzweigtes Alkylthio mit 1 bis 8 Kohlenstoffatomen, geradkettiges oder verzweigtes Halogenalkylthio mit 1 bis 6 Kohlenstoffatomen und 1 bis 5 gleichen oder verschiedenen Halogenatomen, geradkettiges oder verzweigtes Alkylsulfinyl mit 1 bis 8 Kohlenstoffatomen, geradkettiges oder verzweigtes Halogenalkylsulfinyl mit 1 bis 6 Kohlenstoffatomen und 1 bis 5 gleichen oder verschiedenen Halogenatomen, geradkettiges oder verzweigtes Alkylsulfonyl mit 1 bis 8 Kohlenstoffatomen, geradkettiges oder verzweigtes Halogenalkylsulfonyl mit 1 bis 6 Kohlenstoffatomen und 1 bis 5 gleichen oder verschiedenen Halogenatomen, gegebenenfalls einfach bis fünffach, gleichartig oder verschieden durch Halogen
und/oder Alkyl mit 1 bis 4 Kohlenstoffatomen substituiertes Cycloalkyl mit 3 bis 6 Kohlenstoffatomen, für Hydroxycarbonyl, Alkylcarbonyl mit 1 bis 6 Kohlenstoffatomen im geradkettigen oder verzweigten Alkylteil, Alkoxycarbonyl mit 1 bis 6 Kohlenstoffatomen im geradkettigen oder verzweigten Alkoxyteil, Cycloalkylcarbonyl mit 3 bis 6 Kohlenstoffatomen im Cycloalkylteil, Cycloalkoxycarbonyl mit 3 bis 6 Kohlenstoffatomen im Cycloalkylteil, für -Z-R⁶ oder 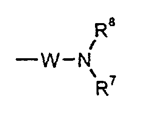 stehen, worin
- R⁶: für Aryl mit 6 bis 10 Kohlenstoffatomen steht, wobei jeder dieser Reste einfach bis dreifach, gleichartig oder verschieden substituiert sein kann durch Halogen, Cyano, Nitro, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen, Alkylsulfinyl mit 1 bis 4 Kohlenstoffatomen, Alkylsulfonyl mit 1 bis 4 Kohlenstoffatomen, Halogenalkylsulfinyl mit 1 bis 4 Kohlenstoffatomen und 1 bis 5 gleichen oder verschiedenen Halogenatomen und/oder Halogenalkylsulfonyl mit 1 bis 4 Kohlenstoffatomen und 1 bis 5 gleichen oder verschiedenen Halogenatomen,
oder
- R⁶: für einen ungesättigten Heterocyclyl-Rest mit 5 oder 6 Ringgliedern und 1 bis 3 Heteroatomen, wie Stickstoff, Sauerstoff und/oder Schwefel steht, wobei diese Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 3 Kohlenstoffatomen im Alkoxyteil, Cycloalkyl mit 3 bis 6 Kohlenstoffatomen, Cyano und/oder Nitro,
- Z: für eine direkte Bindung, sowie für -CH₂-, O, S, SO₂ oder CO steht, oder
für -CO-O- steht, wobei das Sauerstoffatom mit R⁶ verbunden ist, oder
für -SO₂-O- steht, wobei das Schwefelatom mit R⁶ verbunden ist, oder
für -S-CH₂-SO₂- steht, wobei das Schwefelatom der Thio-Gruppe mit R⁶ verbunden ist, oder
für -NH-SO₂- steht, wobei die Sulfonylgruppe mit R⁶ verbunden ist,
- R⁷ und R⁸: unabhängig voneinander für Wasserstoff, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 5 gleichen oder verschiedenen Halogenatomen, geradkettiges oder verzweigtes Alkoxyalkyl mit 1 bis 4 Kohlenstoffatomen im Alkoxyteil und 1 bis 4 Kohlenstoffatomen im Alkylteil, Alkylcarbonyl mit 1 bis 6 Kohlenstoffatomen im geradkettigen oder verzweigten Alkylteil, Aryl mit 6 bis 10 Kohlenstoffatomen, Arylcarbonyl mit 6 bis 10 Kohlenstoffatomen im Arylteil, Arylsulfonyl mit 6 bis 10 Kohlenstoffatomen, Arylaminocarbonyl mit 6 bis 10 Kohlenstoffatomen im Arylteil oder für Arylmethylsulfonyl mit 6 bis 10 Kohlenstoffatomen im Arylteil stehen, wobei jeder der zuvor genannten Aryl-Reste einfach bis dreifach, gleichartig oder verschieden substituiert sein kann durch Halogen, Cyano, Nitro, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen, Alkylsulfinyl mit 1 bis 4 Kohlenstoffatomen, Alkylsulfonyl mit 1 bis 4 Kohlenstoffatomen, Halogenalkylsulfinyl mit 1 bis 4 Kohlenstoffatomen und 1 bis 5 gleichen oder verschiedenen Halogenatomen und/oder Halogenalkylsulfonyl mit 1 bis 4 Kohlenstoffatomen und 1 bis 5 gleichen oder verschiedenen Halogenatomen,
oder
- R⁷ und R⁸: außerdem auch gemeinsam mit dem Stickstoffatom, an das sie gebunden sind, für einen gegebenenfalls einfach bis dreifach durch Alkyl mit 1 bis 4 Kohlenstoffatomen substituierten heterocyclischen Ring mit 5 oder 6 Ringgliedern stehen, der noch ein Sauerstoffatom oder eine C₁-C₄-Alkyliminogruppe enthalten kann,
und
- W: für eine direkte Bindung, eine Sulfonylgruppe oder eine Carbonyl-Gruppe steht,
und
- R¹ und R², oder R² und R³, oder R³ und R⁴: jeweils auch gemeinsam für eine gegebenenfalls einfach bis sechsfach durch Halogen, Alkyl mit 1 bis 4 Kohlenstoffatomen und/oder Halogenalkyl mit 1 bis 4 Kohlenstoffatomen und 1 bis 5 Halogenatomen substituierte Alkylenkette mit 3 oder 4 Gliedern stehen, in der ein oder zwei (nicht benachbarte) Kohlenstoffatome durch Sauerstoffatome ersetzt sein können,
- R⁵ -: für einen ungesättigten Heterocyclyl-Rest mit 5 oder 6 Ringgliedern und 1 bis 3 Heteroatomen, wie Sauerstoff, Stickstoff und/oder Schwefel steht, wobei diese Reste gegebenenfalls einfach bis dreifach, gleichartig oder verschiedenen substituiert sein können durch Halogen, Cyano, Nitro, Amino, Hydroxy, Formyl, Carboxy, Carbamoyl, Thiocarbamoyl, Alkyl mit 1 bis 4 Kohlenstoffatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen, Halogenalkylthio mit 1 bis 4 Kohlenstoffatomen und 1 bis 5 Halogenatomen, Haloalkylsulfonyl mit 1 bis 4 Kohlenstoffatomen und 1 bis 5 Halogenatomen, Alkylamino mit 1 bis 4 Kohlenstoffatomen, Dialkylamino mit jeweils 1 bis 4 Kohlenstoffatomen in den beiden Alkylketten, Alkylcarbonyl mit 1 bis 4 Kohlenstoffatomen im Alkylteil, Alkylcarbonyloxy mit 1 bis 4 Kohlenstoffatomen im Alkylteil, Alkoxycarbonyl mit 1 bis 4 Kohlenstoffatomen im Alkoxyteil, Alkylsulfonyloxy mit 1 bis 4 Kohlenstoffatomen, Hydroximinoalkyl mit 1 bis 4 Kohlenstoffatomen, Alkoximinoalkyl mit jeweils 1 bis 4 Kohlenstoffatomen in den beiden Alkylketten, Halogenalkoxycarbonyl mit 1 bis 4 Kohlenstoffatomen und 1 bis 5 Halogenatomen im Halogenalkoxyteil, und/oder Cycloalkyl mit 3 bis 6 Kohlenstoffatomen,
und
- Q: für Sauerstoff oder Schwefel steht
gefunden.

Weiterhin wurde gefunden, daß man Sulfonylbenzazolone der Formel (I) erhält, wenn man

Benzazolone der Formel 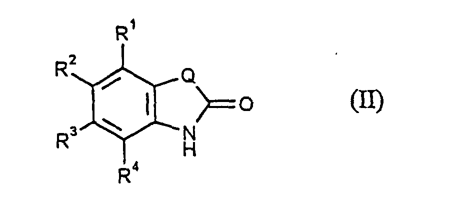 in welcher
- R¹, R², R³, R⁴ und Q: die oben angegebenen Bedeutungen haben,
mit einem Sulfonsäurehalogenid der Formel

R⁵-SO₂-X (III)

in welcher
- R⁵: die oben angegebene Bedeutung hat und
- X: für Halogen steht,
gegebenenfalls in Gegenwart eines Säurebindemittels und gegebenenfalls in Gegenwart eines Verdünnungsmittels umsetzt.

Schließlich wurde gefunden, daß die Sulfonylbenzazolone der Formel (I) sehr gute mikrobizide Eigenschaften besitzen und sowohl im Pflanzenschutz als auch im Materialschutz eingesetzt werden können.

Überraschenderweise zeigen die erfindungsgemäßen Stoffe eine bessere fungizide Wirkung als 7-Nitro-2-oxo-5-trifluormethyl-benzothiazol-3-sulfosäuredimethylamid, welches ein konstitutionell ähnlicher, vorbekannter Wirkstoff gleicher Wirkungsrichtung ist.

Die erfindungsgemäßen Stoffe sind durch die Formel (I) allgemein definiert.
- R¹, R², R³ und R⁴: stehen unabhängig voneinander bevorzugt für Wasserstoff, Fluor, Chlor, Brom, Cyano, Nitro, für Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethoxy, Trifluormethoxy, Difluormethylthio, Trifluormethylthio, Trifluormethylsulfinyl, Trifluormethylsulfonyl, Acetyl, Acetyloxy, Methoxycarbonyl, Ethoxycarbonyl, Cyclopropyl, Cyclobutyl, Cyclopentyl, Cyclohexyl, für -Z-R⁶ oder 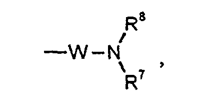
- R⁶: steht bevorzugt für Phenyl, das einfach bis dreifach, gleichartig oder verschieden substituiert sein kann durch Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethoxy, Trifluormethoxy, Difluormethylthio, Trifluormethylthio, Difluormethylsulfinyl und/oder Trifluormethylsulfonyl.
- R⁶: steht auch bevorzugt für Pyrrolyl, Furyl, Thienyl, Pyrazolyl, Imidazolyl, Thiazolyl, Isothiazolyl, Oxazolyl, Isoxazolyl, Oxadiazolyl, Thiadiazolyl, 1,2,3-Triazinyl, 1,2,4-Triazinyl, 1,3,5-Triazinyl, Pyridinyl, Pyrimidinyl, Pyrazinyl oder Pyridazinyl, wobei jeder dieser Reste einfach oder zweifach, gleichartig oder verschieden substituiert sein kann durch Fluor, Chlor, Methyl, Methoxy, Trifluormethyl, Difluormethoxy und/oder Trifluormethoxy.
- Z: steht auch bevorzugt für eine direkte Bindung, sowie für -CH₂-, O, S, SO₂, CO oder
für -CO-O-, wobei das Sauerstoffatom mit R⁶ verbunden ist, oder
für -SO₂-O-, wobei das Schwefelatom mit R⁶ verbunden ist, oder
für -S-CH₂-SO₂-, wobei das Schwefelatom der Thio-Gruppe mit R⁶ verbunden ist, oder
für -NH-SO₂-, wobei die Sulfonylgruppe mit R⁶ verbunden ist.
- R⁷ und R⁸: stehen unabhängig voneinander bevorzugt für Wasserstoff, Methyl, Ethyl, n-Propyl, Isopropyl oder Phenyl.
- R⁷ und R⁸: stehen außerdem auch gemeinsam mit dem Stickstoffatom, an das sie gebunden sind, bevorzugt für Pyrrolidinyl, Piperidinyl, Morpholinyl oder 4-Methyl-piperazinyl.
- W: steht such bevorzugt für eine direkte Bindung, eine Sulfonylgruppe oder eine Carbonyl-Gruppe.
- R¹ und R², oder R² und R³, oder R³ und R⁴: stehen auch gemeinsam bevorzugt für die Gruppierungen -CF₂-O-CF₂-, -O-CF₂-O-, -O-CF₂-CHF-O-, -O-CHF-CHF-O-, -O-CF₂-CF₂-O-, -O-CF₂-CFCl-O- oder -O-CFCl-CFCl-O-.
- R⁵: steht bevorzugt für Pyrrolyl, Furyl, Thienyl, Pyrazolyl, Imidazolyl, Thiazolyl, Isothiazolyl, Oxazolyl, Isoxazolyl, Oxadiazolyl, Thiadiazolyl, 1,2,3-Triazinyl, 1,2,4-Triazinyl, 1,3,5-Triazinyl, Pyridinyl, Pyrimidinyl, Pyrazinyl oder Pyridazinyl, wobei diese Reste einfach, zweifach oder gegebenenfalls auch dreifach gleichartig oder verschieden substituiert sein können durch Fluor, Chlor, Brom, Cyano, Nitro, Amino, Hydroxy, Formyl, Carboxy, Carbamoyl, Thiocarbamoyl, Methyl, Ethyl, n-oder i-Propyl, n-, i-, s- oder t-Butyl, Cyclopropyl, Methoxy, Ethoxy, n-oder i-Propoxy, Methylthio, Ethylthio, n- oder i-Propylthio, Methylsulfonyl, Ethylsulfonyl, Trifluormethyl, Difluormethoxy, Trifluormethoxy, Difluormethylthio, Trifluormethylthio oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroxyiminomethyl, Hydroxyiminoethyl, Methoxyiminomethyl, Ethoxyiminomethyl, Methoxyiminoethyl und/oder Ethoxyiminoethyl.
- Q: steht auch bevorzugt für Sauerstoff oder Schwefel.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte. Als Beispiele für erfindungsgemäße Stoffe seien die in den folgenden Tabellen aufgeführten Sulfonylbenzazolone genannt:

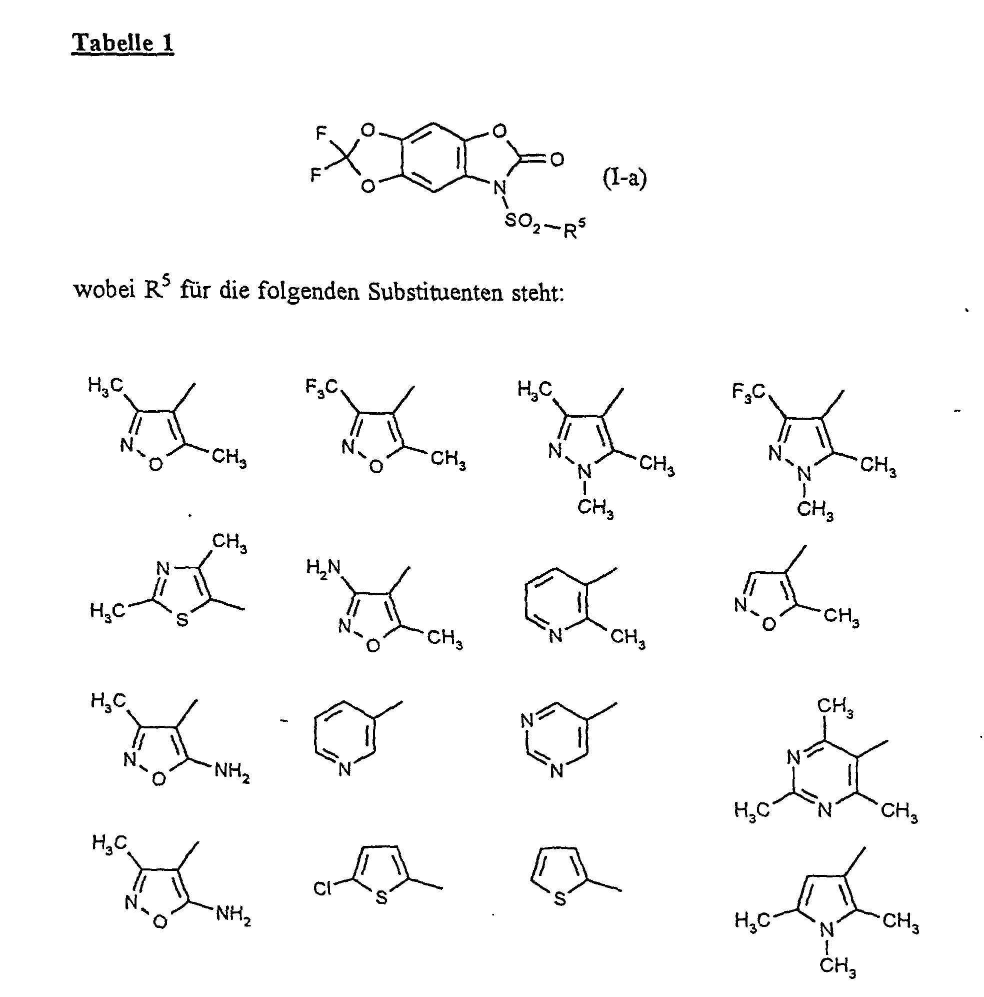

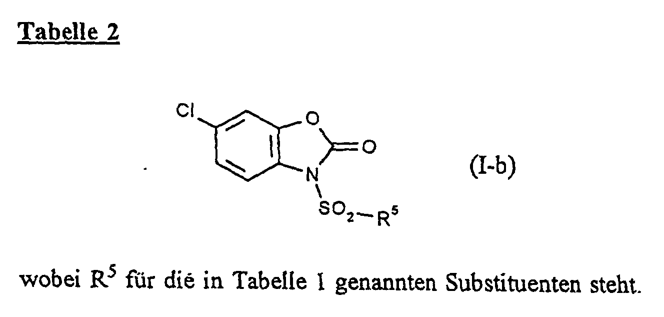

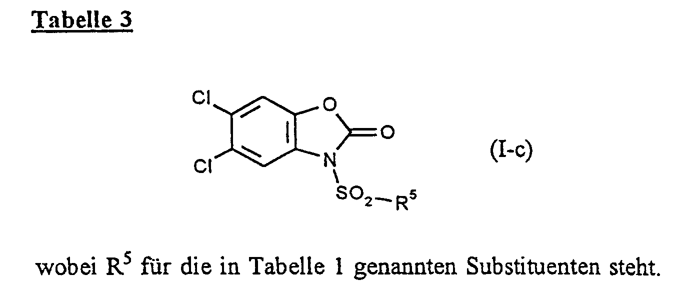

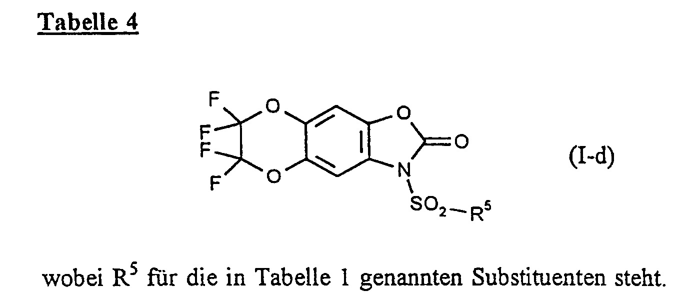

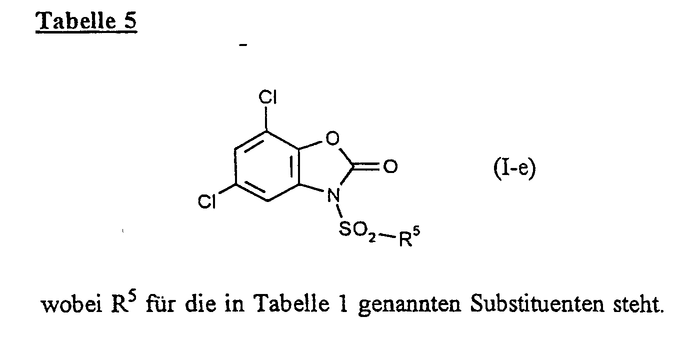

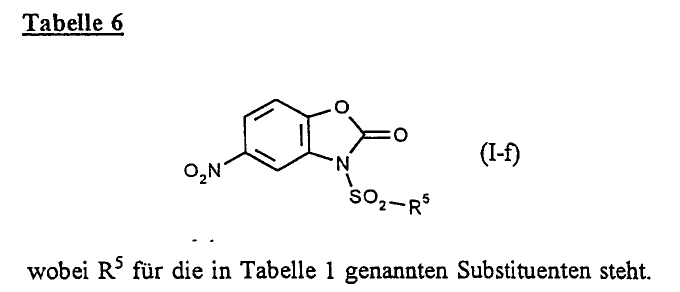

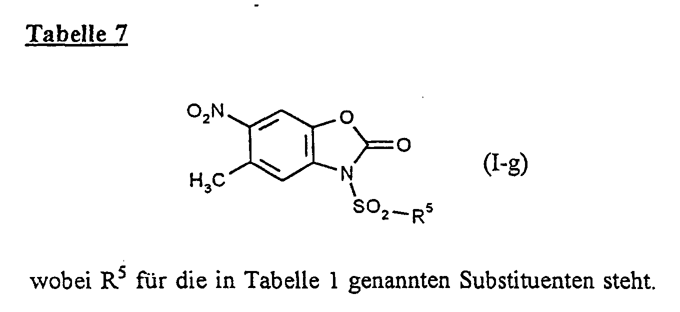

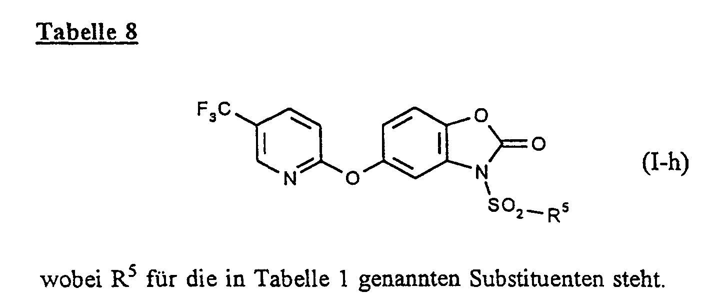

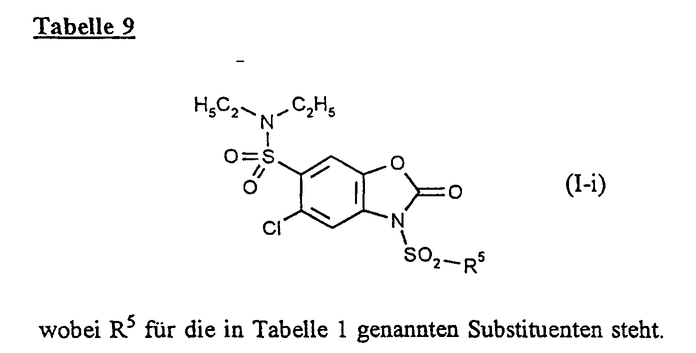

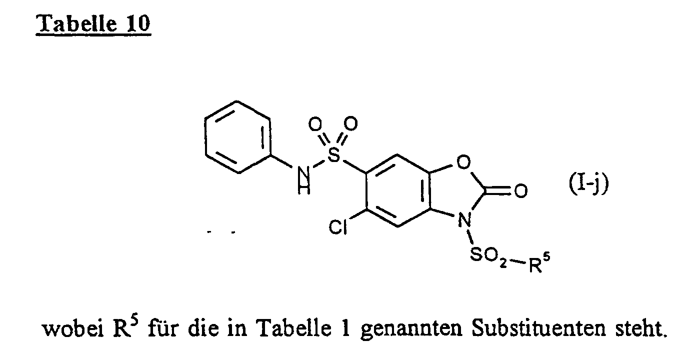

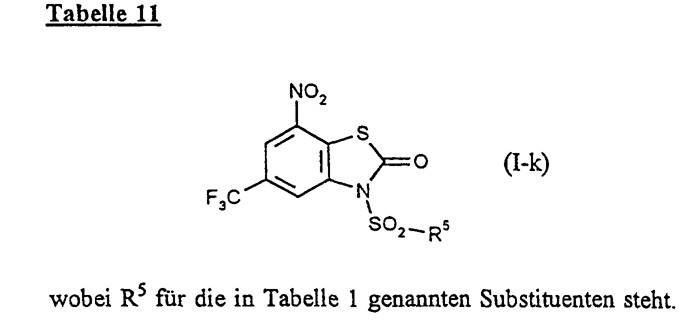

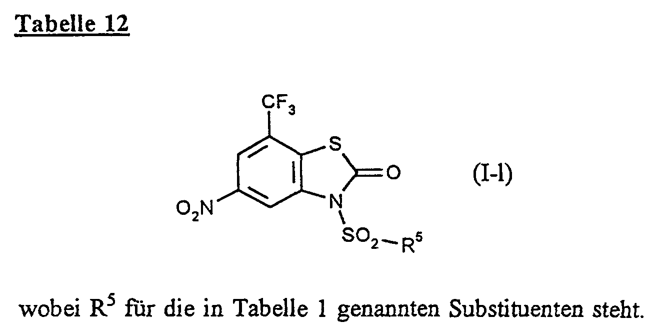

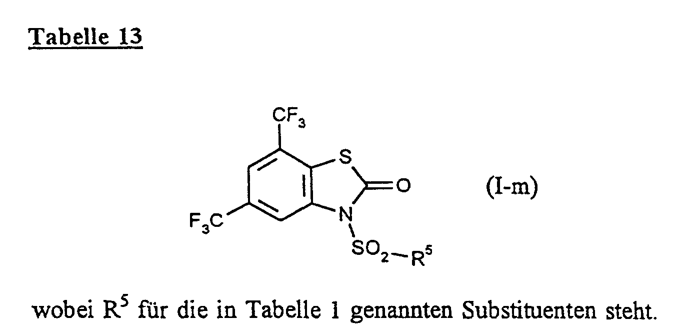

Verwendet man beispielsweise 3H-Benzoxazol-2-on und 3,5-Dimethyl-isoxazol-4-sulfonylchlorid als Ausgangsstoffe, so läßt sich der Verlauf des erfindungsgemäßen Verfahrens durch das folgende Formelschema veranschaulichen:

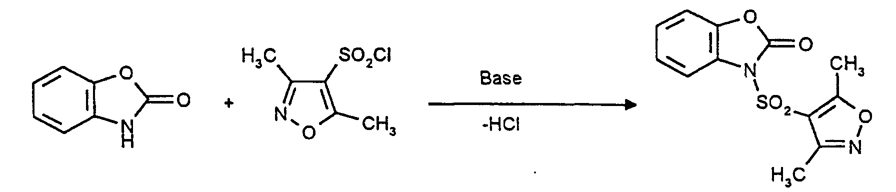

Die zur Durchführung des erfindungsgemäßen Verfahrens als Ausgangstoffe benötigten Benzazolone sind durch die Formel (II) allgemein definiert. In dieser Formel haben R¹, R², R³, R⁴ und Q vorzugsweise diejenigen Bedeutungen, die bereits im Zusammenhang mit der Beschreibung der erfindungsgemäßen Verbindungen der Formel (I) als bevorzugt für R¹, R², R³, R⁴ und Q angegeben wurden.

Die Benzazolone der Formel (II) sind bekannt oder können nach bekannten Methoden hergestellt werden (vgl. Chem. Ber. 93 (1960) 1331-1339; J. Med. Chem. 9 (1966) 719; Heterocycles 24 (1986) 1625; J. Heterocycl. Chem. 28 (1991) 933; Synthesis 1984, 254 und US-A 2 922 794 ).

Die zur Durchführung des erfindungsgemäßen Verfahrens als Reaktionskomponenten weiterhin benötigten Sulfonsäurehalogenide sind durch die Formel (III) allgemein definiert. In dieser Formel hat R⁵ vorzugsweise diejenigen Bedeutungen, die bereits im Zusammenhang mit der Beschreibung der erfindungsgemäßen Verbindungen der Formel (I) als bevorzugt für R⁵ angegeben wurden. X steht vorzugsweise für Chlor.

Die Sulfonsäurehalogenide der Formel (III) sind bekannt oder können nach bekannten Verfahren hergestellt werden (vgl. J. Heterocyclic Chem. 1981, 997-1006).

Als Verdünnungsmittel zur Durchführung des erfindungsgemäßen Verfahrens kommen alle inerten organischen Lösungsmittel in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; ferner halogenierte Kohlenwasserstoffe, wie Chlorbenzol, Dichlorbenzol, Dichlormethan, Chloroform, Tetrachlormethan, Dichlorethan oder Trichlorethan; außerdem Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; weiterhin Ketone, wie Aceton, Butanon, Methyl-isobutylketon oder Cyclohexanon; ferner Nitrile, wie Acetonitril, Propionitril, n- oder i-Butyronitril oder Benzonitril; und auch Ester wie Essigsäuremethylester oder Essigsäureethylester.

Als Säurebindemittel kommen bei der Durchführung des erfindungsgemäßen Verfahrens alle üblichen anorganischen oder organischen Säureakzeptoren in Betracht. Vorzugsweise verwendbar sind Erdalkalimetall- oder Alkalimetallhydride, -hydroxide, -amide, -alkoholate, -acetate, -carbonate oder -hydrogencarbonate, wie Natriumhydrid, Natriumamid, Natrium-methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat, ferner Ammoniumhydroxid, Ammoniumacetat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methyl-piperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 20°C und 120°C.

Bei der Durchführung des erfindungsgemäßen Verfahrens arbeitet man im allgemeinen unter Atmosphärendruck. Es ist aber auch möglich, unter erhöhtem oder vermindertem Druck zu arbeiten, z.B. unter Drucken zwischen 0,1 bar und 10 bar.

Zur Durchführung des erfindungsgemäßen Verfahrens setzt man pro Mol an Benzazolon der Formel (II) im allgemeinen 1 bis 2 Mol, vorzugsweise 1 bis 1,3 Mol, an Sulfonsäurehalogenid der Formel (III) und gegebenenfalls 1,0 bis 2,0 Mol, vorzugsweise 1,0 bis 1,3 Mol an Säureakzeptor ein. Die Aufarbeitung erfolgt nach üblichen Methoden.

Die erfindungsgemäßen Stoffe weisen eine starke mikrobizide Wirkung auf und können zur Bekämpfung von unerwünschten Mikroorganismen, wie Fungi und Bakteria, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Psdeudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen Phytophtora-Arten, einsetzen. Mit gutem Erfolg werden auch Getreidekrankheiten, wie beispielsweise Erysiphe-Arten, oder Reiskrankheiten, wie beispielsweise Pyricularia-Arten, bekämpft. Ferner lassen sich die erfindungsgemäßen Verbindungen auch zur Steigerung des Ernteertrags von Kulturpflanzen einsetzen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe bzw. Mittel gegen Pilze, insbesondere Schimmelpilze, sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole und Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methyl-isobutylketon oder Cyclohexanon, stark polare Lösungsmittel, wie Dimethylformamid oder Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in-Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

### Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticdiin-S, Bromuconazol, Bupirimat, Buthiobat,
Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram, Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilate, Iminoctadinetriacetate, Iodocarb, Ipconazol, Iprobenfos(IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Boerdeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazole, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintocen(PCNB),
Schwefel und Schwefel-Zubereitungen,
Tebuconazol, Tecloftalam, Tecnhazen, Tetcyclacis, Tetraconazol, Thiabendazol, Thicyofen, Thifluzamide, Tthiophanate-methyl, Thiram, Tioxymid, Tolclofosmethyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon, (E)-α(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3 -Triiod-2-propenyl)-2H-tetrazol,
2-[(1-Methylethyl)sulfonyl]-5-(trichlormethyl)-1,3,4-thiadiazol,
2-[[6-Deoxy-.4-.O-(4-O-methyl-β-D-glycopyranosyl)-α-D-glucopyranosyl]-amino]-4-methoxy-1H-pyrrolo[2,3-d]pyrimidin-5-carbonitril, 2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OP),
3,4-Dichlor-1-[4-(difluormethoxy)-phenyl]-1H-pyrrol-2,5-dion,
3,5-Dichlor-N-[cyan-[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-onm,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholin-hydrochlorid,
Ethyl-[(4-chlorphenyl)-azo]-cyanoacetat,
Kaliumhydrogencarbonat,
Methantetrathiol -Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-.oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis(2-propinyloxy)-phenyl]-N'-methoxy-methanimidamid,
N-Formyl-N-hydroxy-DL-alanin -Mono-Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl,-Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.
Auch eine Mischung mit anderen bekannten Wirkstoffen, wie Herbiziden oder mit Düngemitteln und Wachstumsregulatoren ist möglich.

Die Wirkstoffe können als solche, in Form ihrer handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verschäumen, Bestreichen usw.. Es ist ferner möglich die Wirkstoffe nach dem Ultra-Low-Volume-Verfahren auszubringen oder die Wirkstoffzubereitung oder der Wirkstoff selbst in den Boden zu injizieren. Es kann wird auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75%.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-% bezogen auf das zu schützende Material.

Die Herstellung und die Verwendung von erfindungsgemäßen Wirkstoffen werden durch die folgenden Beispiele veranschaulicht.

### Herstellungsbeispiele:

### Beispiel 1

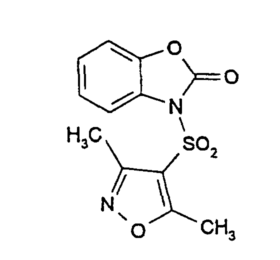

Zu einer Lösung von 2,0 g (15 mmol) 3H-Benzoxazol-2-on in 40 ml absolutem Tetrahydrofuran gibt man 0,45 g (15 mmol) Natriumhydridsuspension (80%ig), rührt 10 Minuten bei 20°C, gibt 3,0 g (15 mmol) 3,5-Dimethylisoxazol-4-sulfonylchlorid hinzu und rührt weitere 20 Stunden bei 20°C. Anschließend gießt man die Reaktionsmischung auf 200 ml Wasser und extrahiert zweimal mit je 50 ml Diethylether. Die vereinigten organischen Phasen werden über Natriumsulfat getrocknet und unter vermindertem Druck eingeengt. Der feste Rückstand wird mit 50 ml Petrolether verrührt, und der erhaltene Feststoff wird abfiltriert und getrocknet.

Man erhält 3,9 g (90% der Theorie) an 3-(3,5-Dimethyl-isoxazol-4-sulfonyl)-3H-benzoxazol-2-on als gelben Feststoff vom Schmelzpunkt 133 bis 135°C.

### Beispiel 2

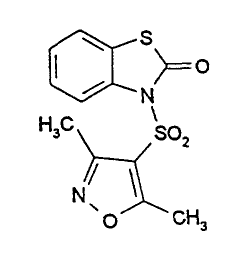

Zu einer Lösung von 2,3 g (15 mmol) 3H-Benzthiazol-2-on in 40 ml absolutem Acetonitril gibt man nacheinander 3,4 g (25 mmol) pulverisiertes Kaliumcarbonat und 2,9 g (15 mmol) 3,5-Dimethylisoxazol-4-sulfonylchlorid und rührt 20 Stunden bei 20°C. Anschließend gießt man die Reaktionsmischung auf 200 ml Wasser, und der erhaltene Feststoff wird abfiltriert und getrocknet.

Man erhält 3,1 g (66% der Theorie) an 3-(3,5-Dimethyl-isoxazol-4-sulfonyl)-3H-benzthiazol-2-on als weißen Feststoff vom Schmelzpunkt 140 bis 144°C.

Nach den zuvor angegebenen Methoden werden auch die in der folgenden Tabelle aufgeführten Stoffe hergestellt.

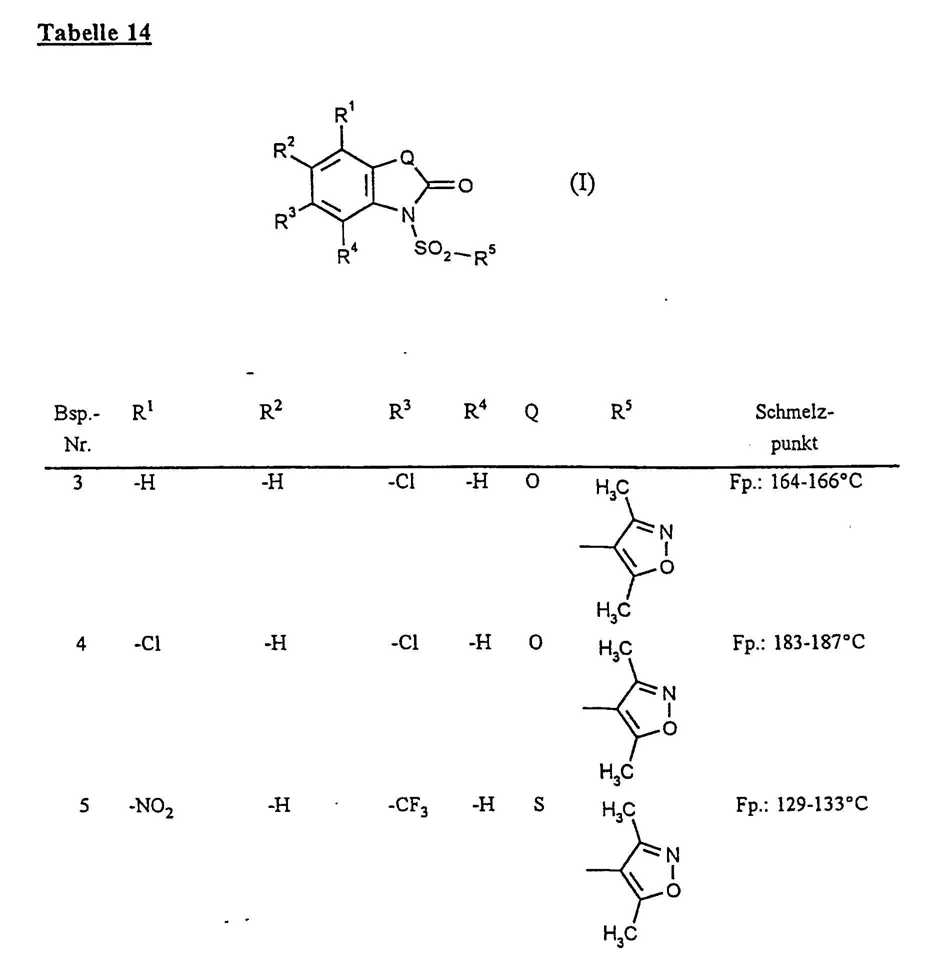

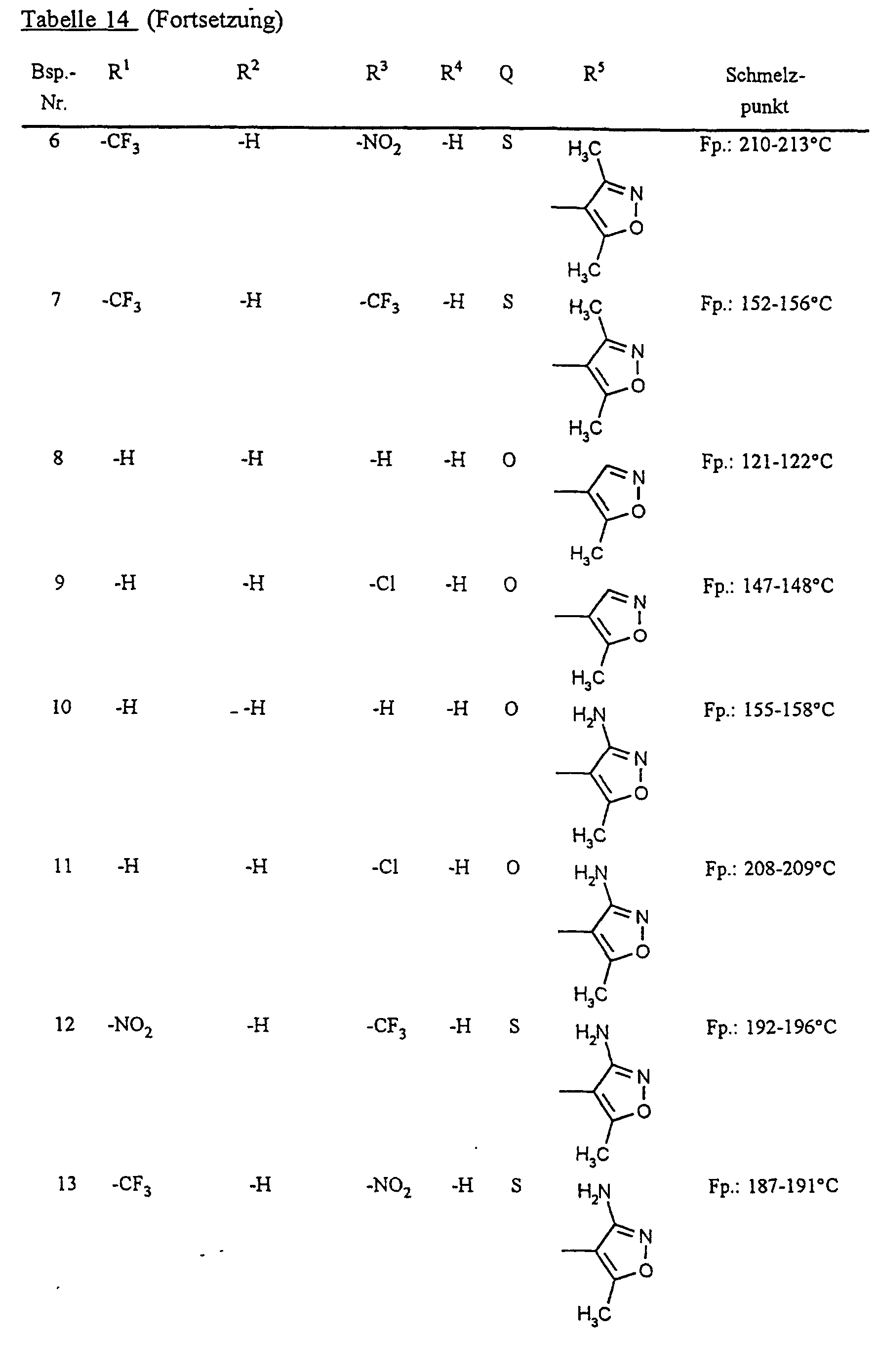

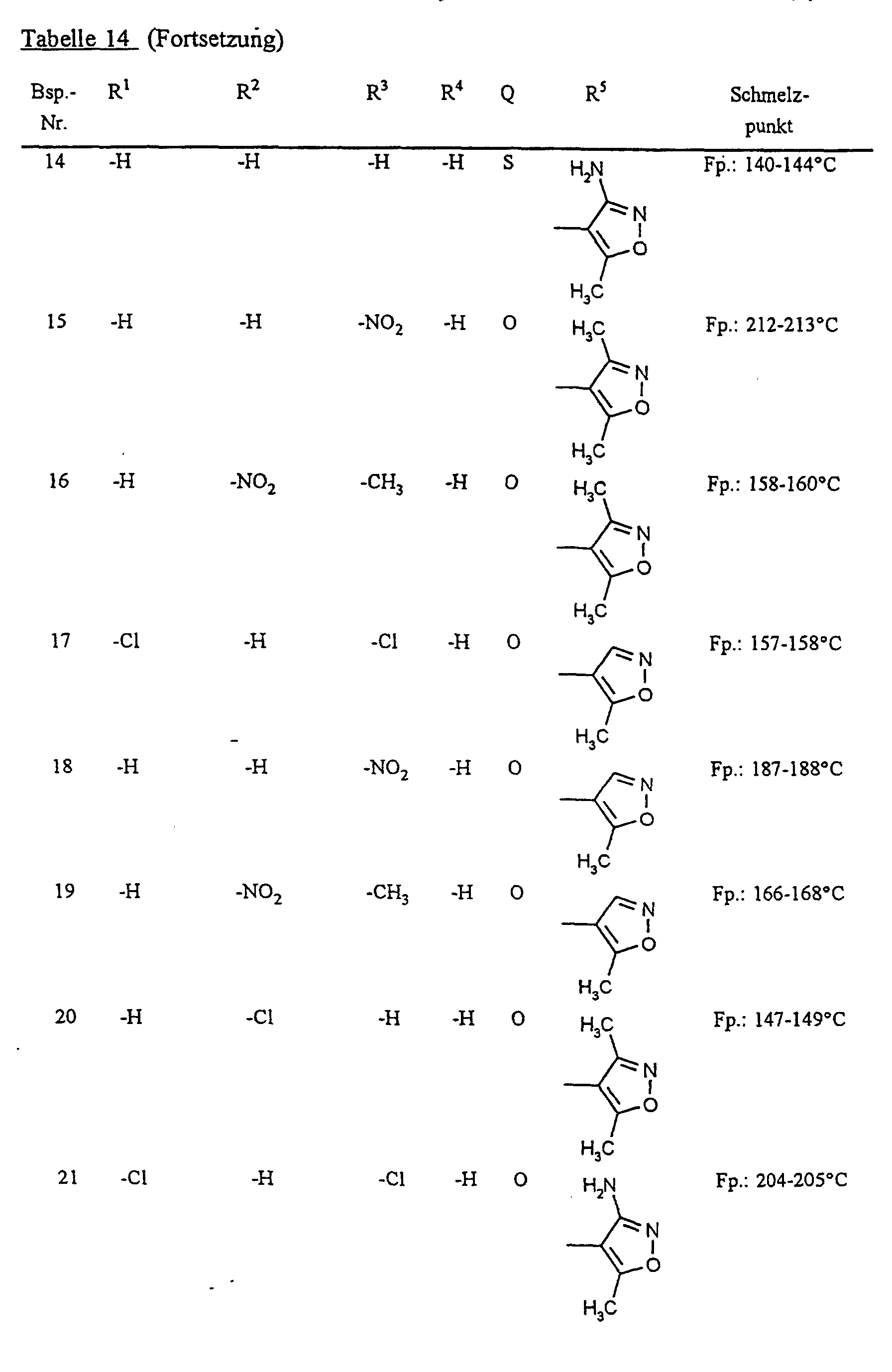

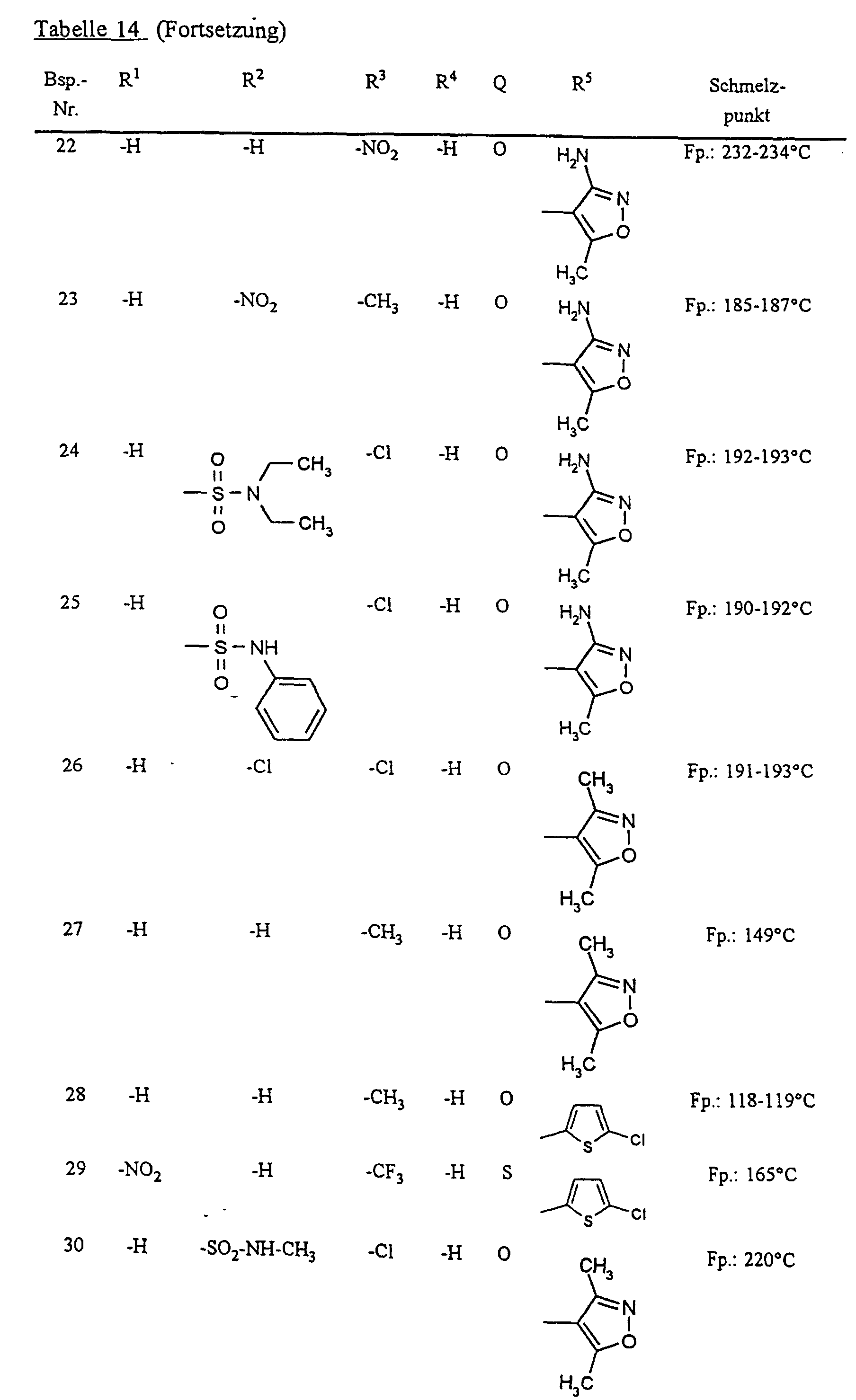

### Verwendungsbeispiele

### Beispiel A

### Phytophthora-Test (Tomate) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von *Phytophthora infestans* inokuliert.

Die Pflanzen werden dann in einer Inkubationskabine bei ca. 20°C und 100% relativer Luftfeuchtigkeit aufgestellt.

3 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Wirkstoffe, Aufwandmengen an Wirkstoffen und Versuchsergebnisse gehen aus der folgenden Tabelle hervor.

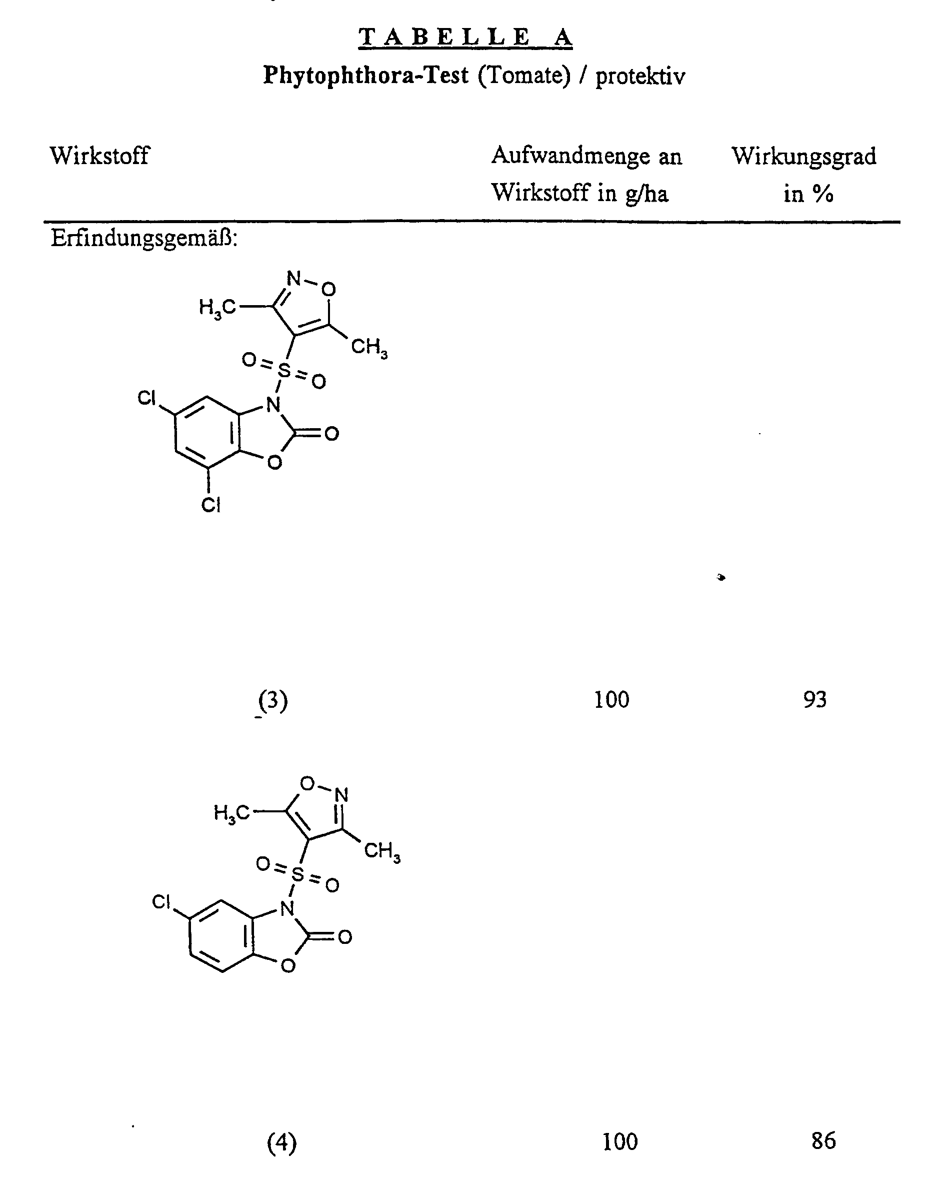

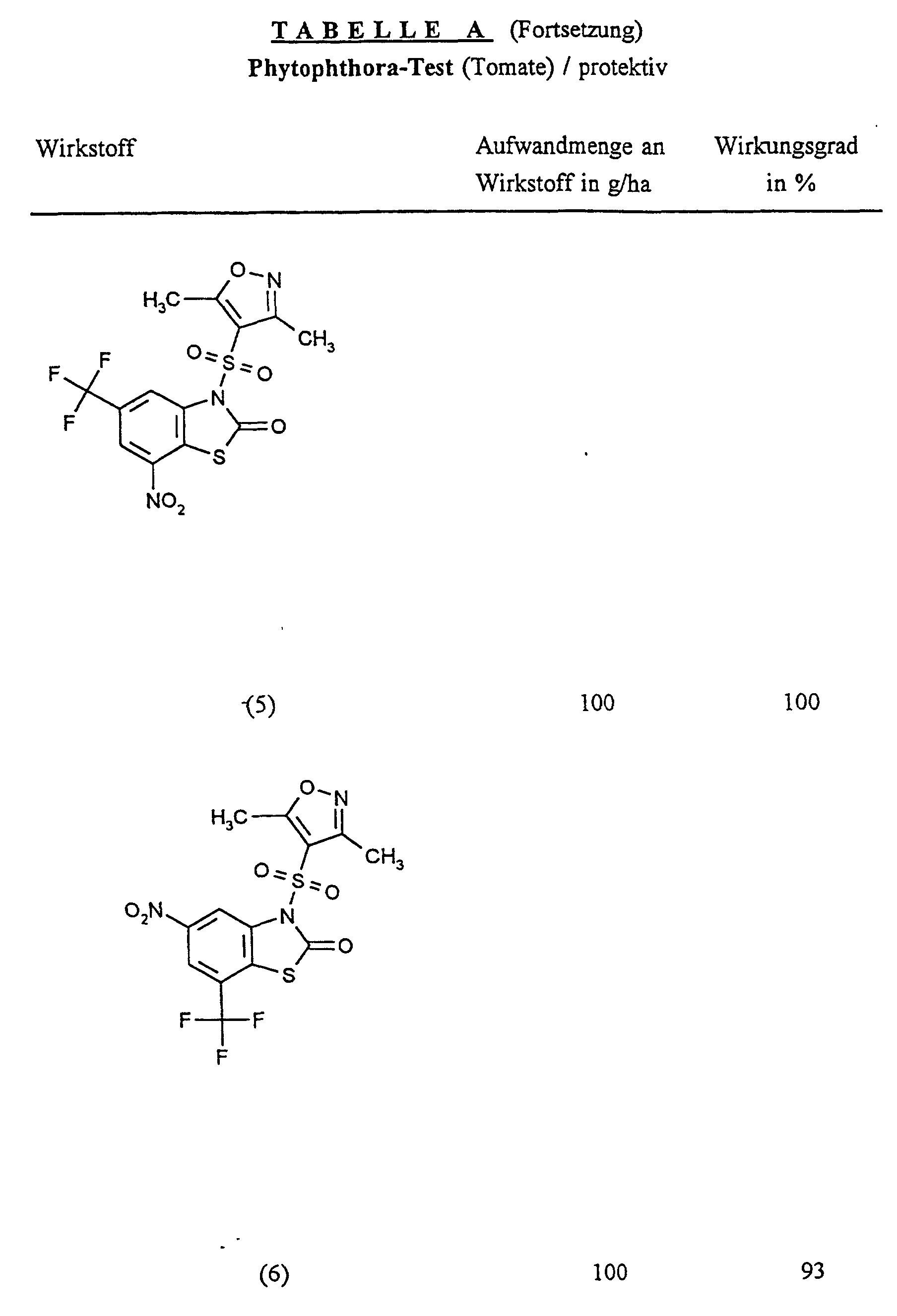

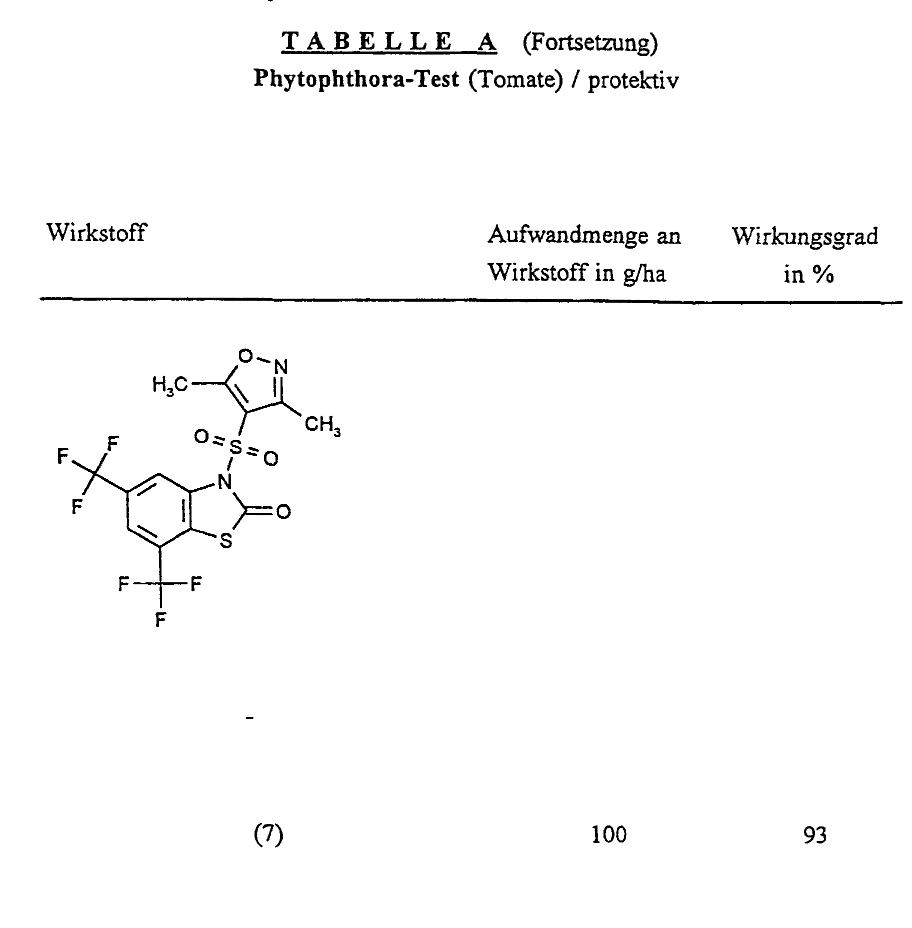

## Patentansprüche

1. Sulfonylbenzazolone der Formel 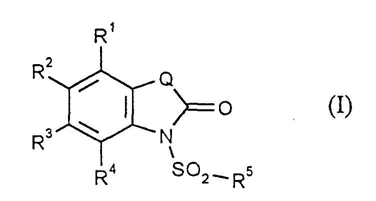 in welcher
R¹, R², R³ und R⁴ unabhängig voneinander für Wasserstoff, Fluor, Chlor, Brom, Iod, Cyano, Nitro, geradkettiges oder verzweigtes Alkyl mit 1 bis 8 Kohlenstoffatomen, geradkettiges oder verzweigtes Halogenalkyl mit 1 bis 6 Kohlenstoffatomen und 1 bis 5 gleichen oder verschiedenen Halogenatomen, geradkettiges oder verzweigtes Alkoxy mit 1 bis 8 Kohlenstoffatomen, geradkettiges oder verzweigtes Halogenalkoxy mit 1 bis 6 Kohlenstoffatomen und 1 bis 5 gleichen oder verschiedenen Halogenatomen, geradkettiges oder verzweigtes Alkylthio mit 1 bis 8 Kohlenstoffatomen, geradkettiges oder verzweigtes Halogenalkylthio mit 1 bis 6 Kohlenstoffatomen und 1 bis 5 gleichen oder verschiedenen Halogenatomen, geradkettiges oder verzweigtes Alkylsulfinyl mit 1 bis 8 Kohlenstoffatomen, geradkettiges oder verzweigtes Halogenalkylsulfinyl mit 1 bis 6 Kohlenstoffatomen und 1 bis 5 gleichen oder verschiedenen Halogenatomen, geradkettiges oder verzweigtes Alkylsulfonyl mit 1 bis 8 Kohlenstoffatomen, geradkettiges oder verzweigtes Halogenalkylsulfonyl mit 1 bis 6 Kohlenstoffatomen und 1 bis 5 gleichen oder verschiedenen Halogenatomen, gegebenenfalls einfach bis fünffach, gleichartig oder verschieden durch Halogen und/oder Alkyl mit 1 bis 4 Kohlenstoffatomen substituiertes Cycloalkyl mit 3 bis 6 Kohlenstoffatomen, für Hydroxycarbonyl, Alkylcarbonyl mit 1 bis 6 Kohlenstoffatomen im geradkettigen oder verzweigten Alkylteil, Alkoxycarbonyl mit 1 bis 6 Kohlenstoffatomen im geradkettigen oder verzweigten Alkoxyteil, Cycloalkylcarbonyl mit 3 bis 6 Kohlenstoffatomen im Cycloalkylteil, Cycloalkoxycarbonyl mit 3 bis 6 Kohlenstoffatomen im Cycloalkylteil, für -Z-R⁶ oder 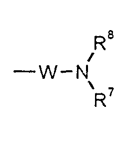 stehen, worin
R⁶ für Aryl mit 6 bis 10 Kohlenstoffatomen steht, wobei jeder dieser Reste einfach bis dreifach, gleichartig oder verschieden substituiert sein kann durch Halogen, Cyano, Nitro, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen, Alkylsulfinyl mit 1 bis 4 Kohlenstoffatomen, Alkylsulfonyl mit 1 bis 4 Kohlenstoffatomen, Halogenalkylsulfinyl mit 1 bis 4 Kohlenstoffatomen und 1 bis 5 gleichen oder verschiedenen Halogenatomen und/oder Halogenalkylsulfonyl mit 1 bis 4 Kohlenstoffatomen und 1 bis 5 gleichen oder verschiedenen Halogenatomen,
oder
R⁶ für einen ungesättigten Heterocyclyl-Rest mit 5 oder 6 Ringgliedern und 1 bis 3 Heteroatomen, wie Stickstoff, Sauerstoff und/oder Schwefel steht, wobei diese Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 3 Kohlenstoff- atomen im Alkoxyteil, Cycloalkyl mit 3 bis 6 Kohlenstoffatomen, Cyano und/oder Nitro,
Z für eine direkte Bindung, sowie für -CH₂-, O, S, SO₂ oder CO steht, oder
für -CO-O- steht, wobei das Sauerstoffatom mit R⁶ verbunden ist, oder
für -SO₂-O- steht, wobei das Schwefelatom mit R⁶ verbunden ist, oder
für -S-CH₂-SO₂- steht, wobei das Schwefelatom der Thio-Gruppe mit R⁶ verbunden ist, oder
für -NH-SO₂- steht, wobei die Sulfonylgruppe mit R⁶ verbunden ist,
R⁷ und R⁸ unabhängig voneinander für Wasserstoff, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 5 gleichen oder verschiedenen Halogenatomen, geradkettiges oder verzweigtes Alkoxyalkyl mit 1 bis 4 Kohlenstoffatomen im Alkoxyteil und 1 bis 4 Kohlenstoffatomen im Alkylteil, Alkylcarbonyl mit 1 bis 6 Kohlenstoffatomen im geradkettigen oder verzweigten Alkylteil, Aryl mit 6 bis 10 Kohlenstoffatomen, Arylcarbonyl mit 6 bis 10 Kohlenstoffatomen im Arylteil, Arylsulfonyl mit 6 bis 10 Kohlenstoffatomen, Arylaminocarbonyl mit 6 bis 10 Kohlenstoffatomen im Arylteil oder für Arylmethylsulfonyl mit 6 bis 10 Kohlenstoffatomen im Arylteil stehen, wobei jeder der zuvor genannten Aryl-Reste einfach bis dreifach, gleichartig oder verschieden substituiert sein kann durch Halogen, Cyano, Nitro, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 - Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen, Alkylsulfinyl mit 1 bis 4 Kohlenstoffatomen, Alkylsulfonyl mit 1 bis 4 Kohlenstoffatomen, Halogenalkylsulfinyl mit 1 bis 4 Kohlenstoffatomen und 1 bis 5 gleichen oder verschiedenen Halogenatomen und/oder Halogenalkylsulfonyl mit 1 bis 4 Kohlenstoffatomen und 1 bis 5 gleichen oder verschiedenen Halogenatomen,
oder
R⁷ und R⁸ außerdem auch gemeinsam mit dem Stickstoffatom, an das sie gebunden sind, für einen gegebenenfalls einfach bis dreifach durch Alkyl mit 1 bis 4 Kohlenstoffatomen substituierten heterocyclischen Ring mit 5 oder 6 Ringgliedern stehen, der noch ein Sauerstoffatom oder eine C₁-C₄-Alkyliminogruppe enthalten kann,
und
W für eine direkte Bindung, eine Sulfonylgruppe oder eine Carbonyl-Gruppe steht,
und
R¹ und R², oder R² und R³, oder R³ und R⁴ jeweils auch gemeinsam für eine gegebenenfalls einfach bis sechsfach durch Halogen, Alkyl mit 1 bis 4 Kohlenstoffatomen und/oder Halogenalkyl mit 1 bis 4 Kohlenstoffatomen und 1 bis 5 Halogenatomen substituierte Alkylenkette mit 3 oder 4 Gliedern stehen, in der ein oder zwei (nicht benachbarte) Kohlenstoffatome durch Sauerstoffatome ersetzt sein können,
R⁵ - für einen ungesättigten Heterocyclyl-Rest mit 5 oder 6 Ringgliedern und 1 bis 3 Heteroatomen, wie Sauerstoff, Stickstoff und/oder Schwefel steht, wobei diese Reste gegebenenfalls einfach bis dreifach, gleichartig oder verschiedenen substituiert sein können durch Halogen, Cyano, Nitro, Amino, Hydroxy, Formyl, Carboxy, Carbamoyl, Thiocarbamoyl, Alkyl mit 1 bis 4 Kohlenstoffatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen, Halogenalkylthio mit 1 bis 4 Kohlenstoffatomen und 1 bis 5 Halogenatomen, Haloalkylsulfonyl mit 1 bis 4 Kohlenstoffatomen und 1 bis 5 Halogenatomen, Alkylamino mit 1 bis 4 Kohlenstoffatomen, Dialkylamino mit jeweils 1 bis 4 Kohlenstoffatomen in den beiden Alkylketten, Alkylcarbonyl mit 1 bis 4 Kohlenstoffatomen im Alkylteil, Alkylcarbonyloxy mit 1 bis 4 Kohlenstoffatomen im Alkylteil, Alkoxycarbonyl mit 1 bis 4 Kohlenstoffatomen im Alkoxyteil, Alkylsulfonyloxy mit 1 bis 4 Kohlenstoffatomen, Hydroximinoalkyl mit 1 bis 4 Kohlenstoffatomen, Alkoximinoalkyl mit jeweils 1 bis 4 Kohlenstoffatomen in den beiden Alkylketten, Halogen-alkoxycarbonyl mit 1 bis 4 Kohlenstoffatomen und 1 bis 5 Halogenatomen im Halogenalkoxyteil, und/oder Cycloalkyl mit 3 bis 6 Kohlenstoffatomen,
und
Q für Sauerstoff oder Schwefel steht.

2. Sulfonylbenzazolon gemäß Anspruch 1, gekennzeichnet durch die Formel

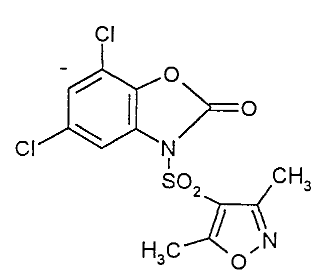

3. Sulfonylbenzazolon gemäß Anspruch 1, gekennzeichnet durch die Formel

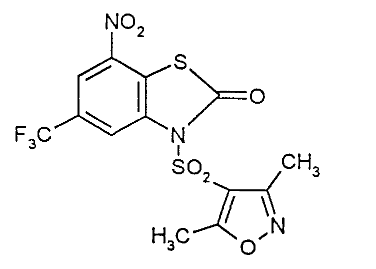

4. Sulfonylbenzazolon gemäß Anspruch 1, gekennzeichnet durch die Formel

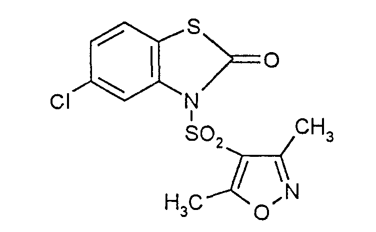

5. Verfahren zur Herstellung von Sulfonylbenzazolonen der Formel (I) gemäß Anspruch 1, dadurch gekennzeichnet, daß man Benzazolone der Formel 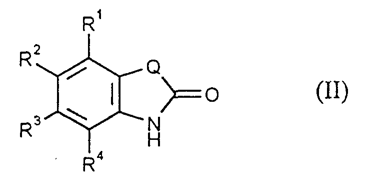 in welcher
R¹, R², R³, R⁴ und Q die oben angegebenen Bedeutungen haben, mit einem Sulfonsäurehalogenid der Formel
R⁵-SO₂-X (III)
in welcher
R⁵ die oben angegebene Bedeutung hat und
X für Halogen steht,
gegebenenfalls in Gegenwart eines Säurebindemittels und gegebenenfalls in Gegenwart eines Verdünnungsmittels umsetzt.

6. Mikrobizide Mittel, gekennzeichnet durch einen Gehalt an mindestens einem Sulfonylbenzazolon der Formel (I) gemäß Anspruch 1 heben Streckmitteln und/oder oberflächen - aktiven Stoffen.

7. Verwendung von Sulfonylbenzazolonen der Formel (I) gemäß Anspruch 1 als Mikrobizide im Pflanzenschutz und im Materialschutz.

8. Verfahren zur Bekämpfung von unerwünschten Mikroorganismen im Pflanzenschutz und im Materialschutz, dadurch gekennzeichnet, daß man Sulfonylbenzazolone der Formel (I) gemäß Anspruch 1 auf die Mikroorganismen und/oder deren Lebensraum ausbringt.

9. Verfahren zur Herstellung von mikrobiziden Mitteln, dadurch gekennzeichnet, daß man Sulfonylbenzazolone der Formel (I) gemäß Anspruch 1 mit Streckmitteln und/oder oberflächenaktiven Stoffen vermischt.

## Claims

1. Sulphonylbenzazolones of the formula 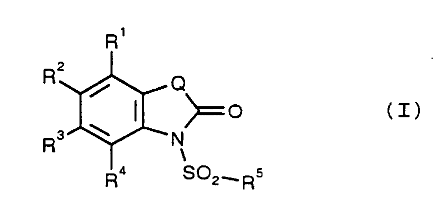 in which
R¹, R², R³ and R⁴ independently of one another each represent hydrogen, fluorine, chlorine, bromine, iodine, cyano, nitro, straight-chain or branched alkyl having 1 to 8 carbon atoms, straight-chain or branched halogenoalkyl having 1 to 6 carbon atoms and 1 to 5 identical or different halogen atoms, straight-chain or branched alkoxy having 1 to 8 carbon atoms, straight-chain or branched halogenoalkoxy having 1 to 6 carbon atoms and 1 to 5 identical or different halogen atoms, straight-chain or branched alkylthio having 1 to 8 carbon atoms, straight-chain or branched halogenoalkylthio having 1 to 6 carbon atoms and 1 to 5 identical or different halogen atoms, straight-chain or branched alkylsulphinyl having 1 to 8 carbon atoms, straight-chain or branched halogenoalkylsulphinyl having 1 to 6 carbon atoms and 1 to 5 identical or different halogen atoms, straight-chain or branched alkylsulphonyl having 1 to 8 carbon atoms, straight-chain or branched halogenoalkylsulphonyl having 1 to 6 carbon atoms and 1 to 5 identical or different halogen atoms, cycloalkyl having 3 to 6 carbon atoms which is optionally mono- to pentasubstituted by identical or different halogens and/or alkyls having 1 to 4 carbon atoms, represents hydroxycarbonyl, alkylcarbonyl having 1 to 6 carbon atoms in the straight-chain or branched alkyl moiety, alkoxycarbonyl having 1 to 6 carbon atoms in the straight-chain or branched alkoxy moiety, cycloalkylcarbonyl having 3 to 6 carbon atoms in the cycloalkyl moiety, cycloalkoxycarbonyl having 3 to 6 carbon atoms in the cycloalkyl moiety, represents -Z-R⁶ or 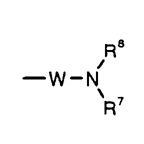 in which
R⁶ represents aryl having 6 to 10 carbon atoms, each of these radicals being mono-to trisubstituted by identical or different substituents from the group consisting of halogen, cyano, nitro, alkyl having 1 to 4 carbon atoms, alkoxy having 1 to 4 carbon atoms, alkylthio having 1 to 4 carbon atoms, halogenoalkyl having 1 to 4 carbon atoms and 1 to 5 identical or different halogen atoms, halogenoalkoxy having 1 to 4 carbon atoms and 1 to 5 identical or different halogen atoms, halogenoalkylthio having 1 to 4 carbon atoms and 1 to 5 identical or different halogen atoms, alkylsulphinyl having 1 to 4 carbon atoms, alkylsulphonyl having 1 to 4 carbon atoms, halogenoalkylsulphinyl having 1 to 4 carbon atoms and 1 to 5 identical or different halogen atoms and/or halogenoalkylsulphonyl having 1 to 4 carbon atoms and 1 to 5 identical or different halogen atoms,
or
R⁶ represents an unsaturated heterocyclyl radical having 5 or 6 ring members and 1 to 3 heteroatoms such as nitrogen, oxygen and/or sulphur, it being possible for these radicals to be mono- to trisubstituted by identical or different substituents from the group consisting of halogen, alkyl having 1 to 4 carbon atoms, alkoxy having 1 to 4 carbon atoms, halogenoalkyl having 1 to 4 carbon atoms and 1 to 5 identical or different halogen atoms, halogenoalkoxy having 1 to 4 carbon atoms and 1 to 5 identical or different halogen atoms, alkoxycarbonyl having 1 to 3 carbon atoms in the alkoxy moiety, cycloalkyl having 3 to 6 carbon atoms, cyano and/or nitro,
Z represents a direct bond, and also represents -CH₂-, O, S, SO₂ or CO or
represents -CO-O- where the oxygen atom is linked to R⁶, or
represents -SO₂-O- where the sulphur atom is linked to R⁶, or
represents -S-CH₂-SO₂- where the sulphur atom of the thio group is linked to R⁶, or
represents -NH-SO₂- where the sulphonyl
group is linked to R⁶,
R⁷ and R⁸ independently of one another each represent hydrogen, straight-chain or branched alkyl having 1 to 6 carbon atoms, straight-chain or branched halogenoalkyl having 1 to 6 carbon atoms and 1 to 5 identical or different halogen atoms, straight-chain or branched alkoxyalkyl having 1 to 4 carbon atoms in the alkoxy moiety and 1 to 4 carbon atoms in the alkyl moiety, alkylcarbonyl having 1 to 6 carbon atoms in the straight-chain or branched alkyl moiety, aryl having 6 to 10 carbon atoms, arylcarbonyl having 6 to 10 carbon atoms in the aryl moiety, arylsulphonyl having 6 to 10 carbon atoms, arylaminocarbonyl having 6 to 10 carbon atoms in the aryl moiety or represents arylmethylsulphonyl having 6 to 10 carbon atoms in the aryl moiety, it being possible for each of the abovementioned aryl radicals to be mono-to trisubstituted by identical or different substituents from the group consisting of halogen, cyano, nitro, alkyl having 1 to 4 carbon atoms, alkoxy having 1 to 4 carbon atoms, alkylthio having 1 to 4 carbon atoms, halogenoalkyl having 1 to 4 carbon atoms and 1 to 5 identical or different halogen atoms, halogenoalkoxy having 1 to 4 carbon atoms and 1 to 5 identical or different halogen atoms, halogenoalkylthio having 1 to 4 carbon atoms and 1 to 5 identical or different halogen atoms, alkylsulphinyl having 1 to 4 carbon atoms, alkylsulphonyl having 1 to 4 carbon atoms, halogenoalkylsulphinyl having 1 to 4 carbon atoms and 1 to 5 identical or different halogen atoms and/or halogenoalkylsulphonyl having 1 to 4 carbon atoms and 1 to 5 identical or different halogen atoms,
or
R⁷ and R⁸ together with the nitrogen atom to which they are attached additionally also represent a heterocyclic ring having 5 or 6 ring members which may contain an additional oxygen atom or a C₁-C₄-alkylimino group and which is optionally mono- to trisubstituted by alkyl having 1 to 4 carbon atoms,
and
W represents a direct bond, a sulphonyl group or a carbonyl group,
and
R¹ and R², or R² and R³, or R³ and R⁴ together each represent an alkylene chain having 3 or 4 members in which one or two (non-adjacent) carbon atoms may be replaced by oxygen atoms and which is optionally mono- to hexasubstituted by halogen, alkyl having 1 to 4 carbon atoms and/or halogenoalkyl having 1 to 4 carbon atoms and 1 to 5 halogen atoms,
R⁵ represents an unsaturated heterocyclyl radical having 5 or 6 ring members and 1 to 3 heteroatoms such as oxygen, nitrogen and/or sulphur, it being possible for these radicals to be optionally mono- to trisubstituted by identical or different substituents from the group consisting of halogen, cyano, nitro, amino, hydroxyl, formyl, carboxyl, carbamoyl, thiocarbamoyl, alkyl having 1 to 4 carbon atoms, alkoxy having 1 to 4 carbon atoms, alkylthio having 1 to 4 carbon atoms, alkylsulphonyl having 1 to 4 carbon atoms, halogenoalkyl having 1 to 4 carbon atoms and 1 to 5 halogen atoms, halogenoalkoxy having 1 to 4 carbon atoms and 1 to 5 halogen atoms, halogenoalkylthio having 1 to 4 carbon atoms and 1 to 5 halogen atoms, halogenoalkylsulphonyl having 1 to 4 carbon atoms and 1 to 5 halogen atoms, alkylamino having 1 to 4 carbon atoms, dialkylamino having 1 to 4 carbon atoms in each of the two alkyl chains, alkylcarbonyl having 1 to 4 carbon atoms in the alkyl moiety, alkylcarbonyloxy having 1 to 4 carbon atoms in the alkyl moiety, alkoxycarbonyl having 1 to 4 carbon atoms in the alkoxy moiety, alkylsulphonyloxy having 1 to 4 carbon atoms, hydroximinoalkyl having 1 to 4 carbon atoms, alkoximinoalkyl having 1 to 4 carbon atoms in each of the two alkyl chains, halogenoalkoxycarbonyl having 1 to 4 carbon atoms and 1 to 5 halogen atoms in the halogenoalkoxy moiety and/or cycloalkyl having 3 to 6 carbon atoms,
and
Q represents oxygen or sulphur.

2. Sulphonylbenzazolone according to Claim 1, characterized by the formula

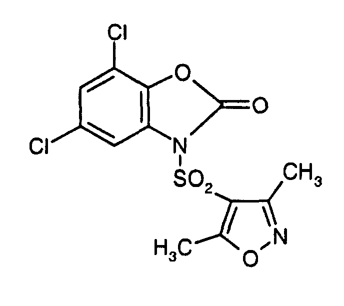

3. Sulphonylbenzazolone according to Claim 1, characterized by the formula

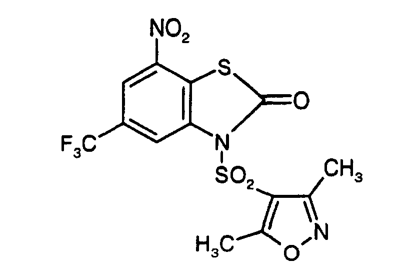

4. Sulphonylbenzazolone according to Claim 1, characterized by the formula

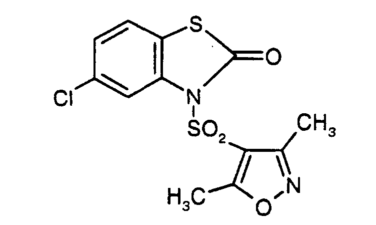

5. Process for preparing sulphonylbenzazolones of the formula (I) according to Claim 1, characterized in that benzazolones of the formula 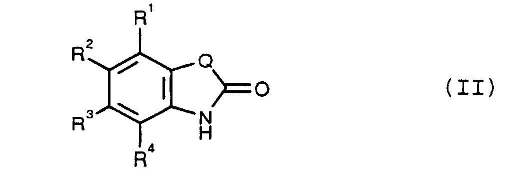 in which
R¹, R², R³, R⁴ and Q are each as defined above are reacted with a sulphonyl halide of the formula
R⁵-SO₂-X (III)
in which
R⁵ is as defined above and
X represents halogen,
if appropriate in the presence of an acid binder and
if appropriate in the presence of a diluent.

6. Microbicidal compositions, characterized by a content of at least one sulphonylbenzazolone of the formula (I) according to Claim 1 in addition to
extenders and/or surfactants.

7. Use the sulphonylbenzazolones of the formula (I) according to Claim 1 as microbicides in crop
protection and in the protection of materials.

8. Method for controlling undesirable microorganisms in crop protection and in the protection of materials, characterized in that sulphonylbenzazolones of the formula (I) according to Claim 1 are applied to the
microorganisms and/or their habitat.

9. Process for preparing microbicidal compositions, characterized in that sulphonylbenzazolones of the formula (I) according to Claim 1 are mixed with extenders and/or surface-active agents.

## Revendications

1. Sulfonylbenzazolones de formule 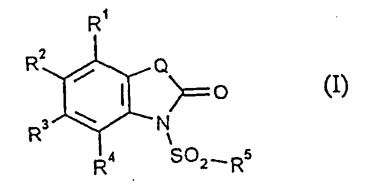 dans laquelle
R¹, R², R³ et R⁴ représentent, indépendamment les uns des autres, l'hydrogène, le fluor, le chlore, le brome, l'iode, un groupe cyano, nitro, alkyle linéaire ou ramifié ayant 1 à 8 atomes de carbone, halogénalkyle linéaire ou ramifié ayant 1 à 6 atomes de carbone et 1 à 5 atomes d'halogènes identiques ou différents, alkoxy linéaire ou ramifié ayant 1 à 8 atomes de carbone, halogénalkoxy linéaire ou ramifié ayant 1 à 6 atomes de carbone et 1 à 5 atomes d'halogènes identiques ou différents, alkylthio linéaire ou ramifié ayant 1 à 8 atomes de carbone, halogénalkylthio linéaire ou ramifié ayant 1 à 6 atomes de carbone et 1 à 5 atomes d'halogènes identiques ou différents, alkylsulfinyle linéaire ou ramifié ayant 1 à 8 atomes de carbone, halogénalkylsulfinyle linéaire ou ramifié ayant 1 à 6 atomes de carbone et 1 à 5 atomes d'halogènes identiques ou différents, alkylsulfonyle linéaire ou ramifié ayant 1 à 8 atomes de carbone, halogénalkylsulfonyle linéaire ou ramifié ayant 1 à 6 atomes de carbone et 1 à 5 atomes d'halogènes identiques ou différents, cycloalkyle de 3 à 6 atomes de carbone éventuellement substitué 1 à 5 fois identiques ou différentes par un halogène et/ou un radical alkyle ayant 1 à 4 atomes de carbone, un groupe hydroxycarbonyle, alkylcarbonyle ayant 1 à 6 atomes de carbone dans la partie alkyle linéaire ou ramifiée, alkoxycarbonyle ayant 1 à 6 atomes de carbone dans la partie alkoxy linéaire ou ramifiée, cycloalkylcarbonyle ayant 3 à 6 atomes de carbone dans la partie cycloalkyle, cycloalkoxycarbonyle ayant 3 à 6 atomes de carbone dans la partie cycloalkyle, un groupe -Z-R⁶ ou 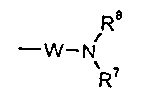 dans lequel
R⁶ représente des restes aryle ayant 6 à 10 atomes de carbone, chacun de ces restes pouvant être substitué 1 à 3 fois identiques ou différentes par un halogène, un radical cyano, nitro, alkyle ayant 1 à 4 atomes de carbone, alkoxy ayant 1 à 4 atomes de carbone, alkylthio ayant 1 à 4 atomes de carbone, halogénalkyle ayant 1 à 4 atomes de carbone et 1 à 5 atomes d'halogènes identiques ou différents, halogénalkoxy ayant 1 à 4 atomes de carbone et 1 à 5 atomes d'halogènes identiques ou différents, halogénalkylthio ayant 1 à 4 atomes de carbone et 1 à 5 atomes d'halogènes identiques ou différents, alkylsulfinyle ayant 1 à 4 atomes de carbone, alkylsulfonyle ayant 1 à 4 atomes de carbone, halogénalkylsulfinyle ayant 1 à 4 atomes de carbone et 1 à 5 atomes d'halogènes identiques ou différents et/ou halogénalkylsulfonyle ayant 1 à 4 atomes de carbone et 1 à 5 atomes d'halogènes identiques ou différents, ou bien
R⁶ représente un reste hétérocyclyle non saturé à noyau pentagonal ou hexagonal ayant 1 à 3 hétéroatomes tels qu'azote, oxygène et/ou soufre, ces restes pouvant être substitués une à trois fois identiques ou différentes par un radical halogéno, alkyle ayant 1 à 4 atomes de carbone, alkoxy ayant 1 à 4 atomes de carbone, halogénalkyle ayant 1 à 4 atomes de carbone et 1 à 5 atomes d'halogènes identiques ou différents, halogénalkoxy ayant 1 à 4 atomes de carbone et 1 à 5 atomes d'halogènes identiques ou différents, alkoxycarbonyle ayant 1 à 3 atomes de carbone dans la partie alkoxy, cycloalkyle ayant 3 à 6 atomes de carbone, cyano et/ou nitro,
Z représente une liaison directe, ainsi que -CH₂-, O, S, SO₂ ou CO, ou bien
un groupe -CO-O-, dont l'atome d'oxygène est lié à R⁶, ou bien
un groupe -SO₂-O-, dont l'atome de soufre est lié à R⁶, ou bien
un groupe -S-CH₂-SO₂-, dans lequel l'atome de soufre du groupe thio est lié à R⁶, ou bien
un groupe -NH-SO₂-, dont le groupe sulfonyle est lié à R⁶,
R⁷ et R⁸ représentent, indépendamment l'un de l'autre, l'hydrogène, un groupe alkyle linéaire ou ramifié ayant 1 à 6 atomes de carbone, un groupe halogénalkyle linéaire ou ramifié ayant 1 à 6 atomes de carbone et 1 à 5 atomes d'halogènes identiques ou différents, un groupe alkoxyalkyle linéaire ou ramifié ayant 1 à 4 atomes de carbone dans la partie alkoxy et 1 à 4 atomes de carbone dans la partie alkyle, un groupe alkylcarbonyle ayant 1 à 6 atomes de carbone dans la partie alkyle linéaire ou ramifiée, un groupe aryle ayant 6 à 10 atomes de carbone, arylcarbonyle ayant 6 à 10 atomes de carbone dans la partie aryle, arylsulfonyle ayant 6 à 10 atomes de carbone, arylaminocarbonyle ayant 6 à 10 atomes de carbone dans la partie aryle ou arylméthyl-sulfonyle ayant 6 à 10 atomes de carbone dans la partie aryle, chacun des restes aryle mentionnés ci-dessus pouvant être substitué une à trois fois identiques ou différentes par un radical halogéno, cyano, nitro, alkyle ayant 1 à 4 atomes de carbone, alkoxy ayant 1 à 4 atomes de carbone, alkylthio ayant 1 à 4 atomes de carbone, halogénalkyle ayant 1 à 4 atomes de carbone et 1 à 5 atomes d'halogènes identiques ou différents, halogénalkoxy ayant 1 à 4 atomes de carbone et 1 à 5 atomes d'halogènes identiques ou différents, halogénalkylthio ayant 1 à 4 atomes de carbone et 1 à 5 atomes d'halogènes identiques ou différents, alkylsulfinyle ayant 1 à 4 atomes de carbone, alkylsulfonyle ayant 1 à 4 atomes de carbone, halogénalkylsulfinyle ayant 1 à 4 atomes de carbone et 1 à 5 atomes d'halogènes identiques ou différents et/ou halogénalkylsulfonyle ayant 1 à 4 atomes de carbone et 1 à 5 atomes d'halogènes identiques ou différents,
ou bien
R⁷ et R⁸ forment aussi en outre, conjointement avec l'atome d'azote auquel ils sont liés, un noyau hétérocyclique pentagonal ou hexagonal éventuellement substitué une à trois fois par un radical alkyle ayant 1 à 4 atomes de carbone et pouvant encore contenir un atome d'oxygène ou un groupe alkylimino en C₁ à C₄,
et
W représente une liaison directe, un groupe sulfonyle ou un groupe carbonyle,
et
R¹ et R², ou bien R² et R³, ou bien R³ et R⁴ forment aussi ensemble dans chaque cas une chaîne alkylène de trois ou quatre chaînons éventuellement substituée une à six fois par un halogène, un radical alkyle ayant 1 à 4 atomes de carbone et/ou un radical halogénalkyle ayant 1 à 4 atomes de carbone et 1 à 5 atomes d'halogènes et dans laquelle un ou deux atomes de carbone (non voisins) peuvent être remplacés par des atomes d'oxygène,
R⁵ représente un reste hétérocyclyle non saturé à noyau pentagonal ou hexagonal ayant 1 à 3 hétéroatomes tels qu'oxygène, azote et/ou soufre, ces restes pouvant éventuellement être substitués une à trois fois identiques ou différentes par un radical halogéno, cyano, nitro, amino, hydroxy, formyle, carboxy, carbamoyle, thiocarbamoyle, alkyle ayant 1 à 4 atomes de carbone, alkoxy ayant 1 à 4 atomes de carbone, alkylthio ayant 1 à 4 atomes de carbone, alkylsulfonyle ayant 1 à 4 atomes de carbone, halogénalkyle ayant 1 à 4 atomes de carbone et 1 à 5 atomes d'halogènes, halogénalkoxy ayant 1 à 4 atomes de carbone et 1 à 5 atomes d'halogènes, halogénalkylthio ayant 1 à 4 atomes de carbone et 1 à 5 atomes d'halogènes, halogénalkylsulfonyle ayant 1 à 4 atomes de carbone et 1 à 5 atomes d'halogènes, alkylamino ayant 1 à 4 atomes de carbone, dialkylamino ayant 1 à 4 atomes de carbone dans chacune des deux chaînes alkyle, alkylcarbonyle ayant 1 à 4 atomes de carbone dans la partie alkyle, alkylcarbonyloxy ayant 1 à 4 atomes de carbone dans la partie alkyle, alkoxycarbonyle ayant 1 à 4 atomes de carbone dans la partie alkoxy, alkylsulfonyloxy ayant 1 à 4 atomes de carbone, hydroximinoalkyle ayant 1 à 4 atomes de carbone, alkoximinoalkyle ayant 1 à 4 atomes de carbone dans chacune des deux chaînes alkyle, halogénalkoxycarbonyle ayant 1 à 4 atomes de carbone et 1 à 5 atomes d'halogènes dans la partie halogénalkoxy, et/ou cycloalkyle ayant 3 à 6 atomes de carbone,
et
Q représente l'oxygène ou le soufre.

2. Sulfonylbenzazolone suivant la revendication 1,
caractérisée par la formule

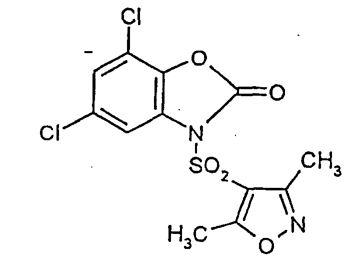

3. Sulfonylbenzazolone suivant la revendication 1,
caractérisée par la formule

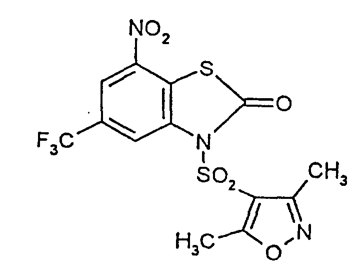

4. Sulfonylbenzazolone suivant la revendication 1,
caractérisée par la formule

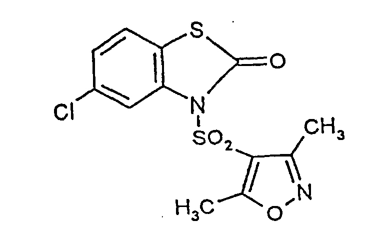

5. Procédé de production de sulfonylbenzazolones de formule (I) suivant la revendication 1, caractérisé en ce qu'on fait réagir des benzazolones de formule 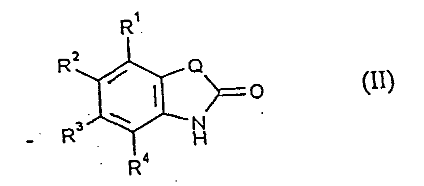 dans laquelle
R¹, R², R³, R⁴ et Q ont les définitions indiquées ci-dessus, avec un halogénure d'acide sulfonique de formule
R⁵-SO₂-X (III)
dans laquelle
R⁵ a la définition indiquée ci-dessus et
X représente un halogène,
le cas échéant en présence d'un accepteur d'acide et éventuellement en présence d'un diluant.

6. Compositions microbicides, caractérisées par une teneur en au moins une sulfonylbenzazolone de formule (I) suivant la revendication 1, à côté de diluants et/ou d'agents tensio-actifs.

7. Utilisation de sulfonylbenzazolones de formule (I) suivant la revendication 1 comme microbicides dans la protection des plantes et la protection des matériaux.

8. Procédé pour combattre des micro-organismes indésirables dans la protection des plantes et dans la protection des matériaux, caractérisé en ce qu'on épand des sulfonylbenzazolones de formule (I) suivant la revendication 1 sur les micro-organismes et/ou sur leur milieu.

9. Procédé de préparation de compositions microbicides, caractérisé en ce qu'on mélange des sulfonylbenzazolones de formule (I) suivant la revendication 1 avec des diluants et/ou des agents tensio-actifs.
